# EUROPEAN PATENT SPECIFICATION

(11) **EP 0 551 574 B1**
(45) Date of publication and mention of the grant of the patent: **25.09.1996**
(21) Application number: 92117694.7
(22) Date of filing: 16.10.1992
(51) Int. Cl.: A61F 2/36, A61F 2/28, A61L 27/00

(54) **Composite implant with metallic braid**
Zusammengesetztes Implantat mit metallischem Geflecht
Implant composite avec tresse métallique

(30) Priority: 13.01.1992 US 819722
(43) Date of publication of application: 21.07.1993
(73) Proprietor: Bristol-Myers Squibb Company, New York, N.Y. 10154 (US)
(72) Inventor: Chu, Helen S., Goshen, IN 46526 (US); Devanathan, Thirumalai N.C., Warsaw, IN 46580 (US)
(74) Representative: Mays, Julie

(56) References cited:
- WO-A-90/00374
- FR-A- 2 105 998
- GB-A- 2 216 425
- US-A- 3 662 405

## Description

### BACKGROUND OF THE INVENTION

The invention of this disclosure relates to composite bone implants.

There have been many attempts to improve bone implants by constructing them of composite materials. U.S. Patent 4,714,467 teaches the use of a composite hip stem with a support collar, a core composed of longitudinal carbon fibers, a braided carbon fiber sheath surrounding the core and a polymer matrix impregnating both sets of fibers. The outer surface is then treated to expose some of the fibers to the bony environment. U.S. Patent 4,902,297 teaches a composite implant prosthesis adapted for efficient mass production comprising a composite carbon fiber core, a braided composite carbon fiber casing over the core and an outer polymer casing defining the desired shape of the implant. UK Patent Application GB 2 216 425 A teaches a bone implant having a core of longitudinally extending fibers, a braided fiber sheath, a polymer skin and a pair of porous fiber metal pads secured to the skin. U.S. Patent 4,750,905 teaches a prosthesis with a composite carbon fiber core, a braided carbon fiber sheath over the core and a polymer skin fused to the core and sheath to define the outer prosthesis geometry. This patent discusses the difficulties in bonding a layered implant. This difficulty in forming a bond may occur during the molding process in which a molten polymer is injected around the relatively cool core and sheath. Because of this temperature difference, the interface between the hot and cold layers cools quickly without efficient fusing of the layers. Improved bonding may be achieved by heating the core prior to molding. However, heating the core, especially a composite one, usually results in distortion of the core and a loss of core compaction. Another proposed solution has been to repeatedly dip the core in a polymer and solvent solution, thereby depositing an increasingly thicker polymer layer. This process is laborious at best. In addition to bonding difficulties, prior devices have had the disadvantage of being difficult or impossible to see on a radiogram.

### SUMMARY OF THE INVENTION

It is therefore an object of the present invention to provide a composite bone implant forming a strong initial bond between its various layers. Another object of the invention is to provide a construction that can be further processed to increase the amount of bonding between its layers. It is a further object of the invention to provide a bone implant that can be readily seen on a radiogram. These and other objectives are achieved by a bone implant, as defined in claim 1, comprising a first non-metallic layer, a metal fiber inner layer and a second non-metallic layer.

### BRIEF DESCRIPTION OF THE DRAWINGS

The before mentioned objects and advantages of the present invention are apparent from the following detailed description and the drawings wherein:
FIG. 1 is a side view of a femoral hip prosthesis according to the present invention.
FIG. 2 is a cross sectional view taken along line 2-2 of FIG. 1.

### DETAILED DESCRIPTION OF THE INVENTION

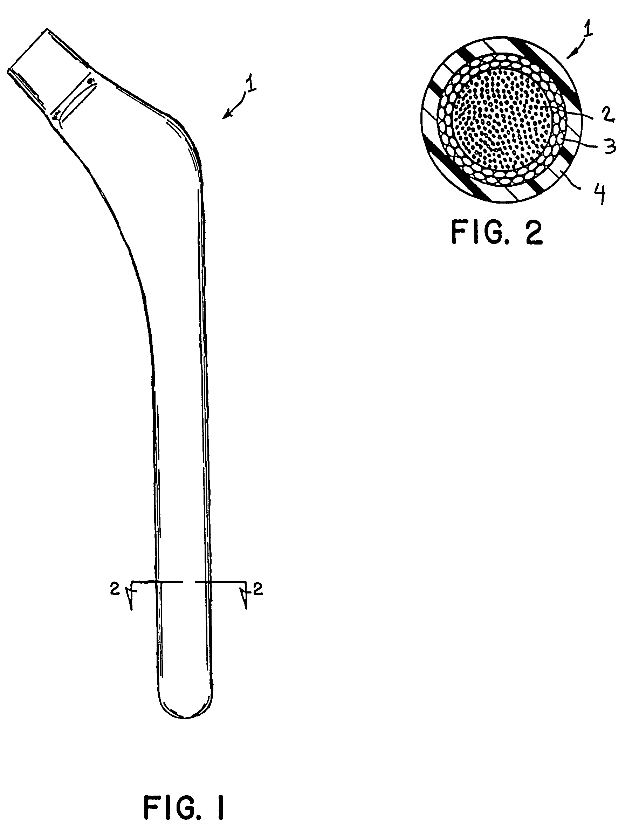

Referring to FIGS. 1 and 2, a preferred embodiment of a femoral hip prosthesis 1 is depicted having a longitudinally oriented composite fiber core first layer 2, a braided metallic sheath inner layer 3 over the core and an outer skin second layer 4. Such a prosthesis can be made by several methods. An exemplary method is disclosed in U.S. Patent 4,902,297 in which a core is formed by drawing polymer impregnated carbon fibers through a die. A sheath of polymer impregnated carbon fibers is then braided over the core. Finally, the core and sheath are placed into an injection molding die and the polymer skin is molded over the core and sheath. It is advantageous to pre-heat the core and sheath prior to the molding step. However, the pre-heat temperature is limited to be below the temperature at which the core will deform or lose compaction or delaminate. For an implant according to the present invention, the braided sheath comprises metal wires. The advantage of using metal wires lies in the mechanical interlock of the polymer with the wire and the further ability of certain polymers to adhere more readily to a wire sheath than to a carbon fiber sheath, thereby imparting enhanced bonding to the layers. The implant is formed so that all of the fibers of the core and sheath are encapsulated and only the polymer skin is exposed to the environment. Polymers of the polyaryletherketone (PAEK) family have the desired adherence to metal as well as appropriate biocompatability for use as an implant. The metal wires can be made of any suitable metal such as stainless steel or titanium alloys. Also, the metal wire layer may be formed by many processes including braiding, winding, weaving or simply matting the fibers together.

In addition to this initially improved bond due to the polymer adhering to the metal, the bond can be further enhanced by induction heating. When an object containing metal is placed in an induction heater, the metal is heated while the non-metallic components remain relatively cool. In induction heater, the metal sheath is heated which in turn heats the polymer at the interface between layers resulting in more complete bonding between layers. Such localized heating is advantageous because it does not result in deformation of the implant as would occur from gross heating of the implant, but it does provide to the interface between layers the extended time at an elevated temperature needed to form an enhanced inter-layer bond.

Finally, apart from improved bond strength, the metal sheath serves to block X-rays so that the implant is visible on a radiogram. This is desirable so that a surgeon may verify implant placement post surgically.

While the foregoing has described a preferred embodiment of the present invention, variations in design and construction are possible. An implant configuration with first and second non-metallic layers and a metallic fiber inner layer between them is within the scope of this invention; whether it be for a hip joint, knee joint, fracture fixation plate or other application.

## Claims

1. A bone implant(1), comprising;
a first non-metallic layer (2);
a second non-metallic layer (4); and
a fibre inner layer (3) disposed between the first (2) and second layers (4), the inner layer (3) and the second layer (4)being securely joined characterised in that the fibres of the inner layer are metallic.

2. A bone implant (1) as claimed in claim 1 characterised in that a bond is formed between two of the layers (2,3,4) by inductively heating the inner layer (3).

3. A bone implant (1) as claimed in any preceding claim characterised in that the metallic fibre inner layer (3) comprises a braided sheath surrounding the first non-metallic layer (2).

4. A bone implant (1) as claimed in any preceding claim characterised in that the first non-metallic layer (2) comprises a thermoplastic.

5. A bone implant (1) as claimed in claim 4 characterised in that the thermoplastic includes a member of the polyaryletherketone family of polymers.

6. A bone implant (1) as claimed in any preceding claim characterised in that the first non-metallic layer (2) further comprises fibres.

7. A bone implant (1) as claimed in any preceding claim characterised in that the first non-metallic layer (2) includes carbon fibres.

8. A bone implant (1) as claimed in any preceding claim characterised in that the bone implant (1) is a femoral hip prosthesis and in that the first non-metallic layer (2) is a core layer, the second non-metallic layer (4) is a skin layer, the inner layer (3) is a metallic fiber sheath layer (3) over the core (2) and the skin layer (4) is formed over the core (2) and sheath (3).

9. A bone implant (1) as claimed in claim 8 characterised in that a bond is formed between two layers (2,3,4) of the femoral hip (1) by inductively heating the inner layer (3).

10. A bone implant (1) as claimed in claim 8 or claim 9 characterised in that the metallic fiber sheath (3) is braided and surrounds the non-metallic core layer (2).

11. A bone implant (1) as claimed in claims 8 to 10 characterised in that the core (2) comprises a thermoplastic and fibres.

12. A bone implant (1) as claimed in claims 8 to 11 characterised in that the core (2) includes a member of the polyaryletherketone family of thermoplastics and the fibres includes carbon.

13. A bone implant (1) as claimed in claims 8 to 12 characterised in that the skin (4) includes a member of the polyaryletherketone family of thermoplastics.

14. A bone implant (1) as claimed in any preceding claim characterised in that the bone implant (1) is a femoral hip stem (1) comprising as the first layer a core (2) including carbon fibers impregnated with a thermoplastic polymer, as the second layer a skin layer (4) including a thermoplastic polymer and as said inner layer a braided metallic fiber sheath (3) enclosing the core (2), the skin layer (4) being formed over the core (2) and sheath (3) such that none of the fibres of the core (2) or sheath (3) are exposed.

## Patentansprüche

1. Knochenimplantat (1) mit:
einer ersten nicht-metallischen Schicht (2);
einer zweiten nicht-metallischen Schicht (4); und
einer Faserinnenschicht (3), die zwischen der ersten (2) und der zweiten Schicht (4) angeordnet ist, wobei die Innenschicht (3) und die zweite Schicht (4) sicher miteinander verbunden sind, dadurch gekennzeichnet, daß die Fasern der Innenschicht metallisch sind.

2. Knochenimplantat (1) nach Anspruch 1, dadurch gekennzeichnet, daß eine Verbindung zwischen zwei von den Schichten (2, 3, 4) durch induktives Heizen der Innenschicht (3) erzeugt wird.

3. Knochenimplantat (1) nach einem der vorstehenden Ansprüche, dadurch gekennzeichnet, daß die metallische Faserinnenschicht (3) eine geflochtene die erste nicht-metallische Schicht (2) umgebende Hülle aufweist.

4. Knochenimplantat (1) nach einem der vorstehenden Ansprüche, dadurch gekennzeichnet, daß die erste nicht-metallische Schicht (2) einen Thermoplast aufweist.

5. Knochenimplantat (1) nach Anspruch 4, dadurch gekennzeichnet, daß der Thermoplast ein Mitglied der Polyaryletherketon-Familie von Polymeren aufweist.

6. Knochenimplantat (1) nach einem der vorstehenden Ansprüche, dadurch gekennzeichnet, daß die erste nicht-metallische Schicht (2) ferner Fasern aufweist.

7. Knochenimplantat (1) nach einem der vorstehenden Ansprüche, dadurch gekennzeichnet, daß die erste nicht-metallische Schicht (2) ferner Kohlefasern aufweist.

8. Knochenimplantat (1) nach einem der vorstehenden Ansprüche, dadurch gekennzeichnet, daß das Knochenimplantat (1) eine Oberschenkel/Hüft-Prothese ist und daß die erste nicht-metallische Schicht (2) eine Kernschicht ist, die zweite nicht-metallische Schicht (4) eine Hautschicht ist, die Innenschicht (3) ein metallische Faserhüllenschicht (3) über dem Kern (2) ist und die Hautschicht (4) über dem Kern (2) und der Hülle (3) ausgebildet ist.

9. Knochenimplantat (1) nach Anspruch 8, dadurch gekennzeichnet, daß eine Verbindung zwischen zwei Schichten (2, 3, 4) der Oberschenkel/Hüft-Prothese (1) durch induktives Heizen erzeugt wird.

10. Knochenimplantat (1) nach Anspruch 8 oder 9, dadurch gekennzeichnet, daß die metallische Faserhülle (3) geflochten ist und die nicht-metallische Kernschicht (2) umgibt.

11. Knochenimplantat (1) nach Anspruch 8 bis 10, dadurch gekennzeichnet, daß der Kern (2) einen Thermoplast und Fasern aufweist.

12. Knochenimplantat (1) nach Anspruch 8 bis 11, dadurch gekennzeichnet, daß der Kern (2) ein Mitglied der Polyaryletherketon-Familie von Thermoplasten aufweist und die Fasern Kohlenstoff aufweisen.

13. Knochenimplantat (1) nach Anspruch 8 bis 12, dadurch gekennzeichnet, daß die Haut (4) ein Mitglied der Polyaryletherketon-Familie von Thermoplasten aufweist.

14. Knochenimplantat (1) nach einem vorstehenden der vorstehenden Ansprüche, dadurch gekennzeichnet, daß das Knochenimplantat (1) ein Oberschenkel/Hüft-Schaft (1) ist, der als die erste Schicht einen Kern (2), der mit einem thermoplastischen Polymer imprägnierte Kohlefasern aufweist, als die zweite Schicht eine Hautschicht (4), die ein thermoplastisches Polymer aufweist , und als die Innenschicht eine geflochtene metallische Faserhülle (3), die den Kern (2) einschließt, aufweist, wobei die Hautschicht (4) über dem Kern (2) und der Hülle (3) in einer Weise ausgebildet ist, daß weder Fasern des Kerns (2) noch der Hülle (3) freiliegen.

## Revendications

1. Implant (1) d'os, comprenant :
une première couche (2) non métallique ;
une seconde couche (4) non métallique ;
une couche interne (3) de fibres disposée entre les première (2) et seconde (4) couches, la couche interne (3) et la seconde couche (4) étant jointes de façon fixe, caractérisé en ce que les fibres de la couche interne sont métalliques.

2. Implant (1) d'os selon la revendication 1 , caractérisé en ce qu'une liaison est formée entre deux des couches (2, 3, 4) par chauffage par induction de la couche interne (3).

3. Implant (1) d'os selon l'une quelconque des revendications précédentes, caractérisé en ce que la couche interne (3) de fibres métalliques comprend une enveloppe tressée entourant la première couche (2) non métallique.

4. Implant d'os (1) selon l'une quelconque des revendications précédentes, caractérisé en ce que la première couche (2) non métallique comprend un thermoplastique.

5. Implant (1) d'os selon la revendication 4, caractérisé en ce que le thermoplastique inclut un membre de la famille des polyaryléthercétones des polymères.

6. Implant (1) d'os selon l'une quelconque des revendications précédentes, caractérisé en ce que la première couche (2) non métallique comprend, de plus, des fibres.

7. Implant (1) d'os selon l'une quelconque des revendications précédentes, caractérisé en ce que la première couche (2) non métallique inclut des fibres de carbone.

8. Implant (1) d'os selon l'une quelconque des revendications précédentes, caractérisé en ce que l'implant (1) d'os est une prothèse de hanche fémorale et en ce que la première couche (2) non métallique est une couche de coeur, la seconde couche (4) non métallique est une couche de peau, la couche interne (3) est une couche (3) d'enveloppe de fibres métalliques sur le coeur (2) et la couche de peau (4) est formée sur le coeur (2) et l'enveloppe (3).

9. Implant (1) d'os selon la revendication 8, caractérisé en ce qu'une liaison est formée entre deux couches (2, 3,4) de la hanche (1) fémorale par chauffage par induction de la couche interne (3).

10. Implant (1) d'os selon la revendication 8 ou la revendication 9, caractérisé en ce que l'enveloppe (3) de fibres métalliques est tressée et entoure la couche (2) de coeur non métallique.

11. Implant (1) d'os selon l'une des revendications 8 à 10, caractérisé en ce que le coeur (2) comprend un thermoplastique et des fibres.

12. Implant (1) d'os selon l'une des revendications 8 à 11, caractérisé en ce que le coeur (2) inclut un membre de la famille des polyaryléthercétones des thermoplastiques et en ce que les fibres incluent du carbone.

13. Implant (1) d'os selon l'une des revendications 8 à 12, caractérisé en ce que la peau (4) inclut un membre de la famille des polyaryléther cétones des thermoplastiques.

14. Implant (1) d'os selon l'une quelconque des revendications précédentes, caractérisé en ce que l'implant (1) d'os est un tronc de hanche fémorale (1) comprenant, en tant que première couche, un coeur (2) incluant des fibres de carbone imprégnées avec un polymère thermoplastique, en tant que seconde couche une couche (4) de peau incluant un polymère thermoplastique et, en tant que ladite couche interne, une enveloppe (3) de fibres métalliques tressées qui enclôt le coeur (2), la couche (4) de peau étant formée sur le coeur (2) et l'enveloppe (3) de sorte qu'aucune des fibres du coeur (2) ou de l'enveloppe (3) ne sont exposées.
